# FASCICULE DE BREVET EUROPEEN

(11) **EP 2 688 680 B1**
(45) Date de publication et mention de la délivrance du brevet: **19.08.2015**
(21) Numéro de dépôt: 12717353.2
(22) Date de dépôt: 22.03.2012
(51) Int. Cl.: B05B 11/00, A61M 15/08

(54) **POMPE DE DISTRIBUTION DE PRODUIT FLUIDE.**
PUMPE FÜR DIE AUSGABE EINES FLÜSSIGPRODUKTS
PUMP FOR DISPENSING A FLUID MATERIAL

(30) Priorité: 25.03.2011 FR 1152488
(43) Date de publication de la demande: 29.01.2014
(73) Titulaire: Aptar France SAS, 27110 Le Neubourg (FR)
(72) Inventeur: PETIT, Ludovic, F-27110 Vitot (FR)
(74) Mandataire: CAPRI
(86) Numéro de dépôt international: PCT/FR2012/050595
(87) Numéro de publication internationale: WO 2012/131234

(56) Documents cités:
- WO-A1-2009/068877
- WO-A2-2010/004224
- FR-A1- 2 838 783

## Description

La présente invention concerne une pompe de distribution de produit fluide et un dispositif de distribution de produit fluide comportant une telle pompe.

Les pompes de distribution de produit fluide sont bien connues dans l'état de la technique, notamment pour distribuer des produits fluides, liquides ou pâteux dans les domaines de la cosmétique, de la parfumerie ou de la pharmacie. Elles comportent généralement un piston coulissant dans un corps de pompe, plus particulièrement dans une chambre de pompe prévue dans ce corps de pompe, et adapté à distribuer une dose de produit fluide à chaque actionnement de la pompe. La chambre de pompe comporte généralement un clapet d'entrée pour permettre de définir la dose de produit expulsée à chaque actionnement. Par ailleurs, notamment avec les produits pharmaceutiques, certaines pompes incorporent parfois des obturateurs au niveau de l'orifice de distribution, pour éviter toute contamination du produit entre deux actionnements.

Un problème qui se pose avec ce type de pompe concerne l'amorçage. En effet, avant le premier actionnement de la pompe, la chambre de pompe est remplie d'air, et il est donc nécessaire d'expulser cet air en totalité pour permettre un remplissage de ladite chambre de pompe avec du produit fluide et permettre un dosage précis et reproductible à chaque actionnement de la pompe. L'amorçage est rendu d'autant plus compliqué que la pompe comporte un obturateur. Il est difficile d'expulser l'air contenu dans la chambre de pompe hors de celle-ci, notamment en raison de la présence dudit obturateur.

Un autre problème qui peut se poser avec les pompes de distribution de produit fluide concerne la qualité du spray, lorsqu'il s'agit d'une pompe de pulvérisation. En effet, particulièrement lorsqu'il y a un obturateur prévu au niveau de l'orifice de distribution, la qualité et les caractéristiques du spray au moment de l'expulsion sont dépendantes dudit obturateur. Or, la plupart de ces obturateurs étant déplacés par la pression du produit créée lors de l'actionnement, des actionnements avec des intensités ou des forces axiales différentes peuvent provoquer des modifications au niveau des caractéristiques du spray. En particulier dans les pompes dans lesquelles l'obturateur se déplace axialement en éloignement de l'orifice de distribution lors de l'actionnement de la pompe, un déplacement trop important dudit obturateur risque de provoquer une perte de qualité du spray, et par conséquent une mauvaise distribution de la dose. De plus, un actionnement avec une force insuffisante peut mener à la distribution d'une dose partielle.

Un autre problème qui peut aussi se poser avec les pompes de distribution de produit fluide concerne le risque de blocage de la pompe. En particulier, ce risque peut survenir avec les pompes à dépression fonctionnant sans reprise d'air extérieur si la dépression dans le réservoir de produit dépasse celle que la chambre de pompe ou de dosage est capable de générer lorsque la pompe revient vers sa position de repos après un actionnement. Le clapet d'entrée de la chambre de pompe peut alors se bloquer et ainsi bloquer la pompe dans son ensemble. Dans ce cas, le pouvoir d'aspiration naturel de la pompe est insuffisant pour contrer la dépression du réservoir, si ce niveau de dépression n'est pas systématiquement retransmis à la chambre de pompe après chaque actionnement. Or, au fur et à mesure qu'une pompe est utilisée, certaines de ses pièces constitutives, réalisées généralement en matière plastique plus ou moins rigide, risquent de se déformer naturellement sous l'effet de la dépression et ainsi sont susceptibles d'engendrer cette augmentation relative désavantageuse de la dépression dans le réservoir par rapport à celle de la chambre de pompe.

Les documents WO 2010/004224 et FR-2 838 783 décrivent des dispositifs de l'art antérieur.

Le document WO 2010/004224 décrit une pompe de distribution de produit fluide selon le préambule de la revendication 1.

La présente invention a pour but de fournir une pompe de distribution de produit fluide qui ne reproduit pas les inconvénients susmentionnés.

Plus particulièrement, la présente invention a pour but de fournir une pompe de distribution de produit fluide qui évite tout risque de blocage de la pompe, notamment en évitant une augmentation excessive de la dépression dans le réservoir par rapport à celle dans la chambre de pompe.

La présente invention a aussi pour but de fournir une pompe de distribution de produit fluide qui permet de réaliser un amorçage sûr et fiable de manière simple et peu coûteuse.

La présente invention a également pour but de fournir une pompe de distribution de produit fluide qui garantit un spray de pulvérisation à chaque actionnement de la pompe, indépendamment de la force que l'utilisateur exerce sur celle-ci lors de son actionnement, et qui garantit la distribution de la totalité de la dose à chaque actionnement.

La présente invention a aussi pour objet de fournir une pompe de distribution de produit fluide qui soit simple et peu coûteuse à fabriquer et à assembler.

La présente invention a donc pour objet une pompe de distribution de produit fluide comportant un premier piston, un second piston et une tête de distribution pourvue d'un orifice de distribution pour actionner ladite pompe, un obturateur étant disposé en amont dudit orifice de distribution, ledit obturateur étant déplaçable entre des positions d'obturation et d'ouverture dudit orifice de distribution, ledit second piston étant formé à l'extérieur d'une pièce creuse coulissant dans ladite tête de distribution, ledit premier piston coulissant à l'intérieur de ladite pièce creuse, ladite pièce creuse comportant une ouverture axiale définie par un bord radial à travers laquelle passe une partie de tige de l'obturateur, ladite partie de tige étant définie entre un épaulement radial proximal et un épaulement radial distal de l'obturateur, ledit obturateur étant déplacé de sa position d'obturation vers sa position d'ouverture par ledit bord radial de ladite pièce creuse qui coopère avec ledit épaulement radial distal, et étant déplacé de sa position d'ouverture vers sa position d'obturation par ledit bord radial qui coopère avec ledit épaulement radial proximal.

Avantageusement, ledit premier piston forme un clapet d'entrée et ledit obturateur forme un clapet de sortie d'une chambre de pompe délimitée par lesdits premier et second pistons.

Avantageusement, pendant l'actionnement, ladite pièce creuse se déplace par rapport à ladite tête de distribution et par rapport audit obturateur.

Avantageusement, ledit obturateur comporte une partie d'obturation qui coulisse dans un manchon disposé dans la tête de distribution en amont de l'orifice de distribution, ledit manchon comportant un profil de pulvérisation.

Avantageusement, un seul ressort sollicite lesdits pistons vers leurs positions de repos et ledit obturateur vers sa position d'obturation.

Avantageusement, ledit ressort est hors de tout contact avec le produit fluide.

Avantageusement, ledit premier piston est réalisé de manière monobloc avec un élément de fixation, tel qu'une bague encliquetable, sertissable ou vissable, adapté à fixer ladite pompe sur un réservoir de produit fluide.

Avantageusement, ledit premier piston est solidaire d'une tige creuse reliée à un tube plongeur s'étendant dans ledit réservoir.

Avantageusement, ladite pièce creuse comporte à proximité dudit bord radial un passage de produit permettant au produit fluide de passer de l'intérieur vers l'extérieur de ladite pièce creuse.

La présente invention a aussi pour objet un dispositif de distribution de produit fluide, comportant un réservoir de produit fluide, et une pompe tel que décrite ci-dessus.

Avantageusement, ladite pompe est fixée sur un réservoir par une bague de fixation encliquetable comportant une jupe périphérique et au moins un profil d'encliquetage saillant radialement vers l'intérieur à partir de ladite jupe périphérique, chaque profil d'encliquetage comportant une paroi axiale espacée de ladite jupe périphérique pour former un espace creux, ledit profil d'encliquetage étant élastiquement déformable dans ledit espace creux.

D'autres caractéristiques et avantages de la présente invention apparaîtront plus clairement au cours de la description détaillée suivante de celle-ci, faite en référence aux dessins joints, donnés à titre d'exemples non limitatifs, et sur lesquels :
- la figure 1 est une vue schématique en section transversale d'une pompe selon un mode de réalisation avantageux de la présente invention, en position de repos ;
- la figure 2 est une schématique en perspective partiellement découpée de la pompe de la figure ;

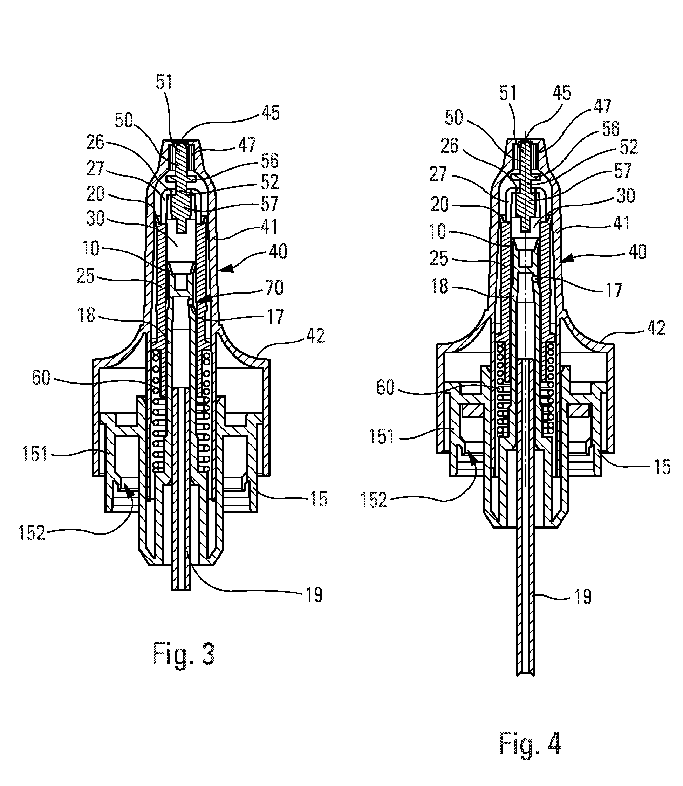

- la figure 3 est une vue similaire à celle de la figure 1, en cours d'actionnement ; et
- la figure 4 est une vue similaire à celle de la figure 1, en fin d'actionnement.

En référence aux figures, un dispositif de distribution de produit fluide comporte une pompe de distribution montée sur un réservoir (non représenté) au moyen d'une bague de fixation 15, avantageusement avec interposition d'un joint d'étanchéité 65. Cette bague de fixation peut être une bague à sertir, à visser ou à encliqueter. Une tête de distribution 40, qui comporte un orifice de distribution 45, est prévue pour actionner ladite pompe 10. Cette tête de distribution peut être une tête nasale, comme représenté sur les dessins, avec une partie axiale allongée 41 destinée à pénétrer dans la narine de l'utilisateur, l'orifice de distribution étant alors à l'extrémité aval de ladite partie allongée de la tête. Une partie d'actionnement élargie 42 est prévue pour permettre à l'utilisateur d'appuyer avec ses doigts sur ladite tête pour actionner la pompe. Avantageusement, l'orifice de distribution est pourvu d'un profil de pulvérisation pour distribuer le produit sous forme de spray finement pulvérisé.

La pompe de distribution comporte un premier piston 10 et un second piston 20, qui coulissent chacun entre des positions de repos et actionnées respectives. Le second piston 20 est formé à l'extérieur d'une pièce creuse 25, et coulisse à l'intérieur de ladite tête de distribution 40. Le premier piston 10 est formé à l'extrémité d'une tige creuse 18 et coulisse à l'intérieur de ladite pièce creuse 25 définissant ledit second piston 20. Le premier piston 10 est fixe par rapport à la bague de fixation 15 et donc par rapport au réservoir. Dans une variante avantageuse représentée sur les dessins, le premier piston 10 est formé de manière monobloc avec ladite bague de fixation 15. La pompe comporte en outre un obturateur 50, disposé directement en amont de l'orifice de distribution 45, et coopérant avec celui-ci en étant déplaçable entre une position d'obturation de l'orifice de distribution 45 et une position d'ouverture de celui-ci. L'orifice de distribution 45 permet de distribuer le produit fluide hors du dispositif, et l'obturateur 50 a pour but de fermer cet orifice entre deux actionnements, notamment pour éviter la contamination du produit fluide contenu dans le dispositif par les contaminants présents dans l'atmosphère.

Avantageusement, la pompe ne comporte qu'un seul ressort 60 adapté à ramener les pistons 10, 20 vers leur positions de repos et l'obturateur 50 vers sa position d'obturation, après chaque actionnement. Ce ressort 60 est avantageusement hors de tout contact avec le produit fluide, ce qui élimine tout risque d'altération du produit fluide en question. Ce ressort 60 coopère avantageusement avec ladite pièce creuse 25 formant le second piston 20. Avantageusement, l'obturateur 50 coulisse axialement entre ses positions d'ouverture et de fermeture dans un manchon 47 prévu dans le fond de la tête d'actionnement 40. Un profil de pulvérisation est de préférence réalisé dans ledit manchon 47 pour permettre un tourbillonnement du produit au moment de son expulsion, pour former un spray. Ainsi, ce profil de pulvérisation n'est pas affecté par le déplacement de l'obturateur vers sa position d'ouverture, ce qui garantit une bonne pulvérisation.

Avantageusement, la tige creuse 18 du premier piston 10 définit un canal s'étendant vers le réservoir. Un tube plongeur 19, destiné à s'étendre jusqu'au fond du réservoir pour distribuer la totalité du produit qu'il contient, peut également être connecté ou réalisé de manière monobloc avec ladite tige creuse 18. Cette tige creuse peut être formée de manière monobloc avec ladite bague de fixation 15. La tige creuse 18 est avantageusement borgne, avec un passage transversal 17 pour permettre au produit qui vient depuis le réservoir de passer de l'intérieur de ladite tige creuse vers son extérieur. La connexion entre la tige creuse 18 et la pièce creuse 25 est étanche en toutes positions, pour éviter des fuites de produit fluide, par exemple vers le ressort 60.

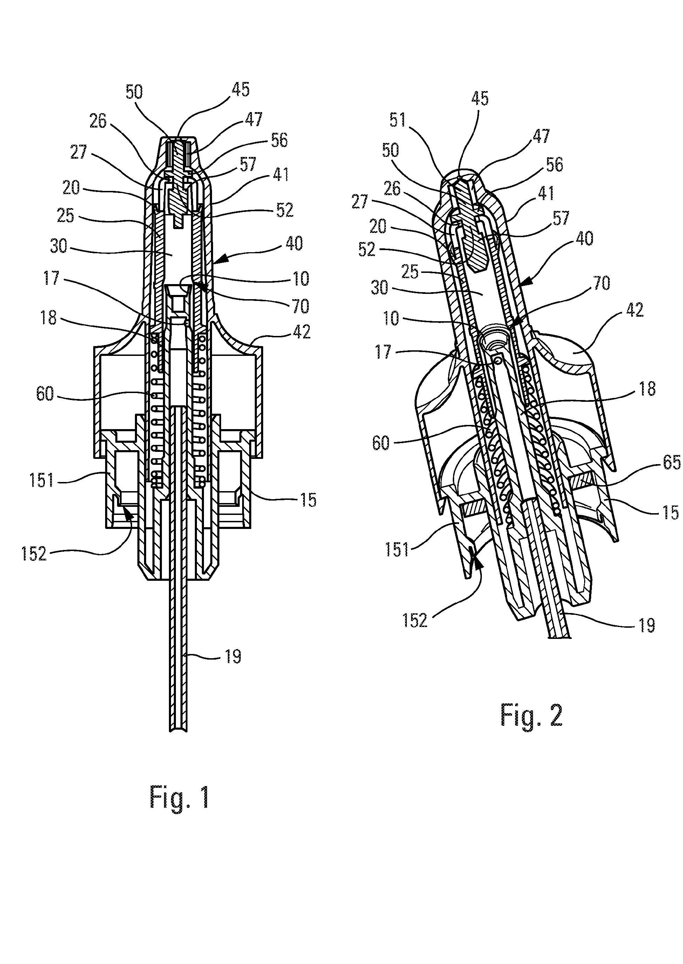

Une chambre de pompe 30 est définie entre lesdits premier et second pistons 10, 20, ledit obturateur 50 et un clapet d'entrée 70. Cette chambre de pompe 30 est donc disposée dans la tête de distribution 40, directement en amont de l'orifice de distribution 45. L'obturateur 50 forme donc le clapet de sortie de la chambre de pompe, en même temps qu'il forme l'élément d'obturation de l'orifice de distribution. Le clapet d'entrée 70 est avantageusement formé par ledit premier piston 10, qui en position d'ouverture dudit clapet d'entrée, coopère de manière non étanche avec une partie de diamètre élargie de ladite pièce creuse 25. En position de repos, visible sur les figures 1 et 2, le premier piston 10 est disposé au niveau de la partie de diamètre agrandie de la pièce creuse 25, de sorte qu'il ne coopère pas de manière étanche avec ladite pièce creuse 25. Le clapet d'entrée 70 est donc ouvert, et le produit venant du réservoir peut donc passer au-delà du premier piston 10. Lorsque le dispositif est actionné, le premier piston 10 va se déplacer dans ladite pièce creuse 25 pour coopérer avec la partie de diamètre réduit, et ainsi fermer le clapet d'entrée 70. A partir de là, le premier piston 10 va agir en tant que piston et coulisser de manière étanche dans ladite pièce creuse 25 jusqu'à sa position d'actionnement. Ce n'est que lorsque le premier piston sera ramené vers sa position de repos que le clapet d'entrée s'ouvrira à nouveau pour permettre le passage de fluide.

La pièce creuse 25 comporte une ouverture axiale supérieure à travers laquelle passe une partie de tige 52 de l'obturateur 50. Cette ouverture axiale définit en outre au moins un passage de produit 27 pour permettre au produit fluide de passer de l'intérieur à l'extérieur de ladite pièce creuse 25. Cette ouverture axiale peut être formée par un trou formé dans la paroi d'extrémité axiale de la pièce creuse 25, de telle sorte que le bord radial 26 de ce trou forme un épaulement radial de ladite pièce creuse. L'obturateur 50 comporte avantageusement une partie d'obturation 51 coopérant avec l'orifice de distribution 45 en position d'obturation. L'obturateur 50 comporte également un épaulement radial proximal 56 et un épaulement radial distal 57, axialement décalés l'un de l'autre, définissant entre eux ladite partie de tige 52. L'épaulement radial proximal 56 est le plus proche de la partie d'obturation 51. Ainsi, comme visible sur les dessins, ledit bord radial 26 peut coulisser autour de ladite partie de tige 52 entre lesdits deux épaulements radiaux 56, 57. Lors de l'actionnement, le bord radial 26 va coopérer en fin d'actionnement avec l'épaulement radial distal 57 pour déplacer ledit obturateur 50 de sa position d'obturation vers sa position d'ouverture. Après actionnement, lorsque le ressort 60 ramène la pièce creuse 25 vers sa position de repos, ledit bord radial 26 va coopérer avec ledit épaulement radial proximal 56 pour ramener ledit obturateur 50 en position d'obturation.

Le fonctionnement normal de la pompe est le suivant. Lorsque l'utilisateur appuie sur la tête de distribution 40, l'épaulement radial proximal 56 de l'obturateur déplace la pièce creuse 25 en comprimant le ressort 60. Le second piston 20 coulisse donc dans ladite tête et le premier piston 10 coulisse à l'intérieur de la pièce creuse 25 pour fermer le clapet d'entrée 70. L'obturateur 50 étant en position d'obturation, la chambre de pompe 30 est donc totalement isolée. Le produit fluide que contient cette chambre de pompe 30 étant incompressible, la poursuite de l'actionnement provoque un déplacement du second piston 20 et donc de la pièce creuse 25 par rapport à ladite tête 40. La pièce creuse 25 va donc descendre à l'intérieur de la tête, avec le bord radial 26 qui va coulisser autour de ladite partie de tige 52 de l'obturateur jusqu'à venir en contact avec l'épaulement radial distal 57. Une poursuite de l'actionnement à partir de là va donc provoquer l'ouverture de l'orifice de distribution 45, l'obturateur 50 étant tiré vers sa position d'ouverture par ledit bord radial 26 coopérant avec ledit épaulement radial distal 57. Cette ouverture de l'obturateur se fait en fin d'actionnement, ce qui empêche toute expulsion de dose partielle due à un actionnement trop faible. Si la force d'actionnement exercée par l'utilisateur est insuffisante, l'obturateur ne s'ouvre pas. A partir du moment que l'obturateur ne s'ouvre qu'en fin de course d'actionnement, la totalité de la dose est distribuée à chaque actionnement. La dose est alors expulsée à travers l'orifice de distribution 45, puis le ressort 60 ramène la pompe en position de repos, ce qui ouvre le clapet d'entrée 70, aspirant ainsi une nouvelle dose de produit fluide dans la chambre de pompe. Avantageusement, en cours d'actionnement, la pression exercée par le produit fluide sur l'épaulement radial proximal 56 de l'obturateur 50 sollicite ce dernier vers sa position d'obturation. Ceci améliore l'efficacité de la pompe en renforçant l'étanchéité pendant l'actionnement.

Lorsque l'utilisateur actionne pour la première fois la pompe et que celle-ci contient de l'air dans la chambre de pompe 30, le clapet d'entrée et l'obturateur sont fermés. L'air étant compressible, le premier piston 10 peut coulisser dans le corps de pompe sans que la pièce creuse 25 ne se déplace par rapport à la tête de distribution 40 et donc sans que l'obturateur 50 ne s'ouvre. Lorsque le premier piston 10 arrive au niveau du passage de produit 27 de la pièce creuse 25, un passage est créé entre la chambre de pompe 30 et le passage transversal 17, ce qui permet d'expulser l'air contenu dans la chambre de pompe 30 vers la tige creuse 18 et donc dans le réservoir. Après amorçage, lorsque la pompe est ramenée vers sa position de repos, du produit fluide est aspiré à l'intérieur de la chambre de pompe 30. Par la suite, le premier piston 10 ne pourra plus atteindre ledit passage de produit 27 de la pièce creuse 25 pendant le fonctionnement normal de la pompe, c'est-à-dire pendant la distribution du produit, mais seulement à la fin de l'actionnement, en position totalement actionnée du premier piston 10.

Le passage transversal 17 et le passage de produit 27 permettent donc de relier la chambre de pompe 20 à la tige creuse 18 et donc au réservoir, lorsque le premier piston 10 atteint sa position totalement actionnée, dans laquelle le premier piston 10 coopère avec le passage de produit 27. Ceci permet d'équilibrer les pressions dans la chambre de pompe 30 et dans le réservoir après chaque actionnement, et ainsi empêche l'augmentation relative de la dépression dans le réservoir par rapport à celle dans la chambre de pompe, ce qui risquerait autrement de bloquer la pompe, par exemple en empêchant l'ouverture du clapet d'entrée 70 de la chambre de pompe.

Selon un aspect avantageux, la bague de fixation 15 est du type encliquetable sur l'extérieur du col de réservoir. Elle comporte à cet effet une jupe périphérique 151 qui est pourvue sur sa surface interne d'au moins un, de préférence plusieurs profils d'encliquetage 152 saillant radialement vers l'intérieur. Avantageusement, plusieurs profils 152 de ce type sont répartis autour de ladite bague 15. Chaque profil d'encliquetage 152 comporte avantageusement une paroi s'étendant en éloignement de ladite jupe périphérique, de préférence de manière inclinée radialement vers le bas (dans la position représentée sur les figures), et dont l'extrémité radialement interne se prolonge par une paroi sensiblement axiale s'étendant vers le bas (dans la position représentée sur les figures). Ainsi, un espace creux est défini entre ladite paroi axiale et la jupe périphérique de la bague, ladite paroi axiale étant espacée de ladite jupe périphérique. Ainsi, chaque profil d'encliquetage 152 est élastiquement déformable dans son espace creux respectif. Cette mise en oeuvre facilite l'encliquetage de la bague 15 sur le col du réservoir, en permettant une déformation des profils d'encliquetage 152. Avantageusement, l'extrémité libre, en l'occurrence l'extrémité inférieure, de la paroi axiale est chanfreinée, ce qui favorise la déformation de ladite paroi axiale dans ledit espace creux lors de l'encliquetage. Cette mise en oeuvre de la bague de fixation 15 facilite aussi sa fabrication par moulage, et notamment le démoulage des profils d'encliquetage 152, qui peut se faire en deux temps. On libère d'abord l'espace creux, ce qui permet ensuite une déformation desdits profils lors du démoulage, sans risquer de les arracher. Il est à noter que cette bague de fixation encliquetable particulière pourrait être utilisée avec tout type de pompe, et que sa mise en oeuvre n'est donc pas nécessairement limitée à la pompe de la présente invention.

L'invention a été décrite en référence à un mode de réalisation particulier de celle-ci, mais il est entendu que diverses modifications pourraient y être apportées. En particulier, les formes de l'obturateur, des pistons, du clapet d'entrée de la chambre de pompe ou des autres éléments, pourraient être réalisées de manière différente si nécessaire. D'autres modifications sont également envisageables pour l'homme du métier sans sortir du cadre de la présente invention tel que défini par les revendications annexées.

## Revendications

1. Pompe de distribution de produit fluide comportant un premier piston (10), un second piston (20) et une tête de distribution (40) pourvue d'un orifice de distribution (45) pour actionner ladite pompe, un obturateur (50) étant disposé en amont dudit orifice de distribution (45), ledit obturateur (50) étant déplaçable entre des positions d'obturation et d'ouverture dudit orifice de distribution (45), ledit second piston (20) étant formé à l'extérieur d'une pièce creuse (25) coulissant dans ladite tête de distribution (40), ledit premier piston (10) coulissant à l'intérieur de ladite pièce creuse (25), **caractérisée en ce que** ladite pièce creuse (25) comporte une ouverture axiale définie par un bord radial (26) à travers laquelle passe une partie de tige (52) de l'obturateur (50), ladite partie de tige (52) étant définie entre un épaulement radial proximal (56) et un épaulement radial distal (57) de l'obturateur (50), ledit obturateur (50) étant déplacé de sa position d'obturation vers sa position d'ouverture par ledit bord radial (26) de ladite pièce creuse (25) qui coopère avec ledit épaulement radial distal (57), et étant déplacé de sa position d'ouverture vers sa position d'obturation par ledit bord radial (26) qui coopère avec ledit épaulement radial proximal (56).

2. Pompe selon la revendication 1, dans laquelle ledit premier piston (10) forme un clapet d'entrée (70) et ledit obturateur (50) forme un clapet de sortie d'une chambre de pompe (30) délimitée par lesdits premier et seconds pistons (10, 20).

3. Pompe selon l'une quelconque des revendications précédentes, dans laquelle, pendant l'actionnement, ladite pièce creuse (25) se déplace par rapport à ladite tête de distribution (40) et par rapport audit obturateur (50).

4. Pompe selon l'une quelconque des revendications précédentes, dans laquelle ledit obturateur (50) comporte une partie d'obturation (51) qui coulisse dans un manchon (47) disposé dans la tête de distribution (40) en amont de l'orifice de distribution (45), ledit manchon (47) comportant un profil de pulvérisation.

5. Pompe selon l'une quelconque des revendications précédentes, dans laquelle un seul ressort (60) sollicite lesdits pistons (10, 20) vers leurs positions de repos et ledit obturateur (50) vers sa position d'obturation.

6. Pompe selon la revendication 5, dans laquelle ledit ressort (60) est hors de tout contact avec le produit fluide.

7. Pompe selon l'une quelconque des revendications précédentes, dans laquelle ledit premier piston (10) est réalisé de manière monobloc avec un élément de fixation (15), tel qu'une bague encliquetable, sertissable ou vissable, adapté à fixer ladite pompe sur un réservoir de produit fluide.

8. Pompe selon l'une quelconque des revendications précédentes, dans laquelle ledit premier piston (10) est solidaire d'une tige creuse (18) reliée à un tube plongeur (19) s'étendant dans ledit réservoir.

9. Pompe selon l'une quelconque des revendications précédentes, dans laquelle ladite pièce creuse (25) comporte à proximité dudit bord radial (26) un passage de produit (27) permettant au produit fluide de passer de l'intérieur vers l'extérieur de ladite pièce creuse (25).

10. Dispositif de distribution de produit fluide, comportant un réservoir de produit fluide, **caractérisé en ce qu'**il comporte une pompe selon l'une quelconque des revendications précédentes.

11. Dispositif de distribution de produit fluide selon la revendication 10, dans lequel ladite pompe est fixée sur un réservoir par une bague de fixation encliquetable (15) comportant une jupe périphérique (151) et au moins un profil d'encliquetage (152) saillant radialement vers l'intérieur à partir de ladite jupe périphérique (151), chaque profil d'encliquetage (152) comportant une paroi axiale espacée de ladite jupe périphérique pour former un espace creux, ledit profil d'encliquetage étant élastiquement déformable dans ledit espace creux.

## Patentansprüche

1. Pumpe für die Ausgabe eines Fluidprodukts, umfassend einen ersten Kolben (10), einen zweiten Kolben (20) und einen Ausgabekopf (40), der mit einer Ausgabeöffnung (45) versehen ist, um die Pumpe zu betätigen, wobei ein Verschluss (50) stromaufwärts der Ausgabeöffnung (45) angeordnet ist, wobei der Verschluss (50) zwischen einer Verschlussposition und einer Öffnungsposition der Ausgabeöffnung (45) verschiebbar ist, wobei der zweite Kolben (20) an der Außenseite eines Hohlstücks (25) gebildet ist, welches in dem Ausgabekopf (40) gleitet, wobei der erste Kolben (10) im Inneren des Hohlstücks (25) gleitet, **dadurch gekennzeichnet, dass** das Hohlstück (25) eine axiale Öffnung umfasst, die durch einen radialen Rand (26) definiert ist, durch welchen ein Stangenteil (52) des Verschlusses (50) führt, wobei das Stangenteil (52) zwischen einem proximalen radialen Absatz (56) und einem distalen radialen Absatz (57) des Verschlusses (50) definiert ist, wobei der Verschluss (50) durch den radialen Rand (26) des Hohlstücks (25), der mit dem distalen radialen Absatz (57) zusammenwirkt, von seiner Verschlussposition in seine Öffnungsposition verschoben wird und durch den radialen Rand (26), der mit dem proximalen radialen Absatz (56) zusammenwirkt, von seiner Öffnungsposition in seine Verschlussposition verschoben wird.

2. Pumpe nach Anspruch 1, wobei der erste Kolben (10) eine Einlassklappe (70) und der Verschluss (50) eine Auslassklappe einer Pumpenkammer (30) bildet, die durch den ersten und den zweiten Kolben (10, 20) begrenzt ist.

3. Pumpe nach einem der vorhergehenden Ansprüche, wobei sich das Hohlstück (25) in Bezug auf den Ausgabekopf (40) und in Bezug auf den Verschluss (50) während der Betätigung verschiebt.

4. Pumpe nach einem der vorhergehenden Ansprüche, wobei der Verschluss (50) ein Verschlussteil (51) umfasst, welches in einer Hülse (47) gleitet, die im Ausgabekopf (40) stromaufwärts der Ausgabeöffnung (45) angeordnet ist, wobei die Hülse (47) ein Sprühprofil umfasst.

5. Pumpe nach einem der vorhergehenden Ansprüche, wobei eine einzige Feder (60) die Kolben (10, 20) in ihre Ruheposition und den Verschluss (50) in seine Verschlussposition belastet.

6. Pumpe nach Anspruch 5, wobei die Feder (60) in keiner Weise mit dem Fluidprodukt in Kontakt ist.

7. Pumpe nach einem der vorhergehenden Ansprüche, wobei der erste Kolben (10) aus einem Stück mit einem Befestigungselement (15), wie einem Rastring, Klemmring oder Schraubring, gefertigt ist, das dazu geeignet ist, die Pumpe auf einem Behälter für ein Fluidprodukt zu befestigen.

8. Pumpe nach einem der vorhergehenden Ansprüche, wobei der erste Kolben (10) mit einer Hohlstange (18) aus einem Stück ist, die mit einem Tauchrohr (19) verbunden ist, das sich in dem Behälter erstreckt.

9. Pumpe nach einem der vorhergehenden Ansprüche, wobei das Hohlstück (25) in der Nähe des radialen Rands (26) einen Produktdurchlass (27) umfasst, der es dem Fluidprodukt erlaubt, vom Inneren zum Äußeren des Hohlstücks (25) zu gelangen.

10. Ausgabevorrichtung für ein Fluidprodukt, umfassend einen Behälter für ein Fluidprodukt, **dadurch gekennzeichnet, dass** sie eine Pumpe nach einem der vorhergehenden Ansprüche umfasst.

11. Ausgabevorrichtung für ein Fluidprodukt nach Anspruch 10, wobei die Pumpe auf einem Behälter durch einen einrastbaren Befestigungsring (15) befestigt ist, der einen Umfangsmantel (151) und mindestens ein Rastprofil (152) umfasst, das radial vom Umfangsmantel (151) nach Innen vorspringt, wobei jedes Rastprofil (152) eine axiale Wand umfasst, die vom Umfangsmantel beabstandet ist, um einen Hohlraum zu bilden, wobei das Rastprofil in dem Hohlraum elastisch verformbar ist.

## Claims

1. A fluid dispenser pump comprising a first piston (10), a second piston (20), and a dispenser head (40), provided with a dispenser orifice (45), for actuating said pump, a shutter (50) being arranged upstream from said dispenser orifice (45), said shutter (50) being movable between closed and open positions of said dispenser orifice (45), said second piston (20) being formed outside a hollow part (25) and being slidable inside said dispenser head (40), said first piston (10) being slidable inside said hollow part (25), said fluid dispenser pump being **characterized in that** said hollow part (25) includes an axial opening that is defined by a radial edge (26) through which there passes a stem portion (52) of the shutter (50), said stem portion (52) being defined between a proximal radial shoulder (56) and a distal radial shoulder (57) of the shutter (50), said shutter (50) being moved from its closed position to its open position by said radial edge (26) of said hollow part (25) co-operating with said distal radial shoulder (57), and being moved from its open position to its closed position by said radial edge (26) co-operating with said proximal radial shoulder (56).

2. A pump according to claim 1, wherein said first piston (10) forms an inlet valve (70), and said shutter (50) forms an outlet valve of a pump chamber (30) that is defined by said first and second pistons (10, 20).

3. A pump according to either preceding claim, wherein, during actuation, said hollow part (25) moves relative to said dispenser head (40) and relative to said shutter (50).

4. A pump according to any preceding claim, wherein said shutter (50) includes a closure portion (51) that slides in a sleeve (47) that is arranged in the dispenser head (40) upstream from the dispenser orifice (45), said sleeve (47) including a spray profile.

5. A pump according to any preceding claim, wherein a single spring (60) urges said pistons (10, 20) towards their rest positions, and said shutter (50) towards its closed position.

6. A pump according to claim 5, wherein said spring (60) does not come into any contact with the fluid.

7. A pump according to any preceding claim, wherein said first piston (10) is made integrally with a fastener element (15), such as a snap-fastenable, crimpable, or screw-fastenable ring, that is adapted to fasten said pump on a fluid reservoir.

8. A pump according to any preceding claim, wherein said first piston (10) is secured to a hollow rod (18) that is connected to a dip tube (19) that extends into said reservoir.

9. A pump according to any preceding claim, wherein, in the proximity of said radial edge (26), said hollow part (25) includes a fluid passage (27), enabling the fluid to pass from the inside to the outside of said hollow part (25).

10. A fluid dispenser device including a fluid reservoir, the device being **characterized in that** it includes a pump according to any preceding claim.

11. A fluid dispenser device according to claim 10, wherein said pump is fastened on a reservoir by a snap-fastener ring (15) that includes a peripheral skirt (151) and at least one snap-fastener profiles (152) that projects radially inwards from said peripheral skirt (151), each snap-fastener profile (152) including an axial wall that is spaced apart from said peripheral skirt so as to form a hollow gap, said snap-fastener profile being elastically deformable into said hollow gap.
